# EUROPEAN PATENT APPLICATION

(11) **EP 0 677 298 A1**
(43) Date of publication of application: **18.10.1995**
(21) Application number: 95400834.8
(22) Date of filing: 12.04.1995
(51) Int. Cl.: A61M 5/32, A61M 5/50

(54) **Disposable device for parenteral injections**

(30) Priority: 13.04.1994 FR 9404688
(71) Applicant: Pince My, Lucette Renée, F-34000 Montpellier (FR)
(72) Inventor: Pince My, Lucette Renée, F-34000 Montpellier (FR)
(74) Representative: Colas, Jean-Pierre

(57) **Abstract**

A device for parenteral injections, comprises a body in which a plunger moves, and a needle which retracts automatically and completely within the body at the end of the injection when the plunger arrives at the base of the body, which contains a movable base integral with the locking end-piece of the needle and is blocked by one or more bolts engaged in a recess in the wall of the body, a spring being held compressed on the lower face of the movable base. The plunger bears a cam which acts directly on the bolt or bolts upon abutment of the plunger against the upper face of the movable base, provoking the unbloking of the movable base, so that the spring can then push back the movable base and the plunger, with the needle coming completely within the body.

## Description

The present invention relates to a disposable device for parenteral injections, the needle of which device retracts automatically and completely within the body of the apparatus at the end of the operation.

Parenteral injection of pharmaceutical substances is traditionally performed with the aid of a "disposable" plastic syringe consisting of a cylindrical body, at the end of which there is fixed a needle which is adapted to the type of injection, and within which cylindrical body a plunger slides. After the injection, the operator has to replace the means of protection (cap) on the needle, detach the needle from the syringe, and place syringe and needle in containers intended to receive these items of medical waste.

The description of this equipment as being "disposable" in fact depends entirely on the sense of responsibility of the users, and a needle and syringe which are possibly contaminated can easily be reused by irresponsible users. Furthermore, the users of this type of equipment are obliged to carry out a large number of handling operations with the needle after injection, resulting in a considerable risk of contamination by accidental injury, a risk which subsequently remains throughout the chain involved in treating this type of medical waste equipment.

To eliminate the risks of possible contamination, US Patent No. 4,973,316, to Edward D. Dysarz, has proposed a device for parenteral injections, comprising a body in which a plunger moves, and a needle which retracts automatically and completely within the body at the end of the injection when the plunger arrives at the base of the body, comprising a movable base which is integral with the locking end-piece of the needle and is blocked by one or more bolts engaged in a recess in the wall of the body, a spring held compressed on the lower face of the movable base, the abutment of the plunger against the upper face of the movable base provoking the release of the bolt or bolts, by displacement of a cam surface, and the unblocking of the movable base, so that the spring can then push back the movable base and the plunger, with the needle coming completely within the body.

In this device, the bolts are moved by cam surfaces which are borne by the movable base. The latter must first be moved forward by the plunger, in order to release the bolts, and it is then pushed back in the opposite direction by the spring. This results in relatively complicated designs of the movable base and of the body. In addition, there is a risk of inadvertent release of the bolts when the liquid to be injected is unusually thick.

Swiss Patent No. 669,910, to Michel-Elie RITZI, describes a similar device, but one which comprises two coaxial tubes: the lower tube contains the "breech" which bears the needle, and the space between the two tubes contains the plunger, which, at the end of its travel, frees catches which pass through the wall of the inner tube. The space in which the needle comes to sit is not the one in which the plunger moves, and the spring does not push back the plunger at the end of the operation.

This device too is complicated and expensive. Its leakproofness at the level of the catches also seems rather doubtful.

The object of the invention is to provide a device which offers a high level of safety, while at the same time being simpler than those of the prior art.

The invention proposes a device for parenteral injections, comprising a body in which a plunger moves, and a needle which retracts automatically and completely within the body at the end of the injection when the plunger arrives at the base of the body, comprising a movable base which is integral with the locking end-piece of the needle and is blocked by one or more bolts engaged in a recess in the wall of the body, a spring held compressed on the lower face of the movable base, the abutment of the plunger against the upper face of the movable base provoking the release of the bolt or bolts, by displacement of a cam surface, and the unblocking of the movable base, so that the spring can then push back the movable base and the plunger, with the needle coming completely within the body. In this device, the plunger bears said cam, which acts directly on the bolt or bolts.

The invention leads to structures which are simpler than those of the prior art, and to a higher level of safety, because the movable base moves in a single direction.

According to particular embodiments, a zone of lesser strength situated along the length of the pusher makes it possible to break the latter level with the body, preventing an accidental escape of the needle, and limiting the size of the equipment as to be disposed.

The release cam can be rectilinear and act in only one plane, the plunger being aligned by a rail fashioned on the push-rod sliding in a guide placed on the body near its opening, or by an oval cross section of the whole syringe; it can be circular and act regardless of its radial orientation on the head of the bolts.

The pusher can be constructed in two parts, the one sliding in the other, these parts being integral during the injection by means of a connection which can be broken by a pressure on the pusher slightly greater than that which is necessary for the release of the bolts, in such a way that the needle can be retracted without the pusher rising.

The spring which pushes the movable base preferably bears on at least one of its end seats at a point which is sufficiently eccentric in relation to the axis of the needle to ensure that, when this spring is compressed after having been released, it assumes the general shape of an arc of a circle and deflects the needle from the axial direction, the deflection being sufficient to ensure that the needle cannot leave the body of the device through the orifice via which it entered.

The attached drawings illustrate the invention:
- Figure 1 represents an axial section of the invention ready to draw up the solution to be injected,
- Figure 2 represents an axial section of the invention at the moment when the retraction of the needle is triggered,
- Figure 3 represents the same section after retraction of the needle.

With reference to these drawings, the device comprises a cylindrical body 1 which bears, in its lower part, two recesses 2 on the inside of the cylinder. Engaged at the bottom of this cylinder is a movable base 3 which is formed by a "flat" cylinder which has a bore 4 running through its thickness along its diameter and whose upper face presents a cylindrical recess in which are placed the heads 5 of two bolts 6, the barrels 7 of which slide, on either side, in said diametral bore 4 which they pass through in order to lock in the recesses 2. The movable base 3 is continued, on its lower face, by a "tall" cylinder of smaller diameter 8 which is equipped, at its end, with a locking cone 9 for an injection needle 10, and which bears a coaxial spring 11 which is held compressed by virtue of the blocking upwards of the movable base 3. The movable base 3 is bored, along its entire height, with an axial channel 12 which permits the passage of the liquid into the needle 10. Situated above this movable base 3 is a cylindrical plunger 13 which is integral with a pusher 14 and which has, at its surface, a cam 15 formed as a conical recess on the section of a cylinder, so that all its axial sections represent a W shape.

The pusher 14 comprises two telescopic parts, which are connected by frangible parts 16.

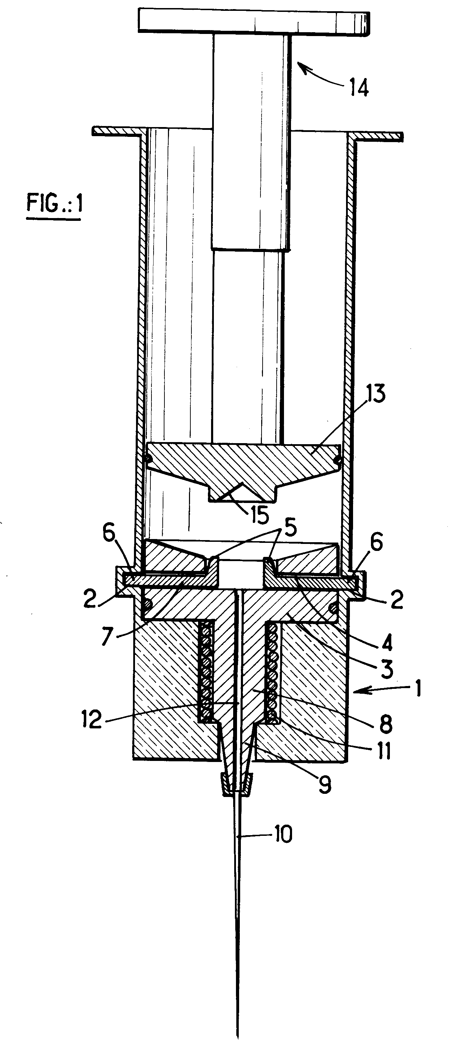

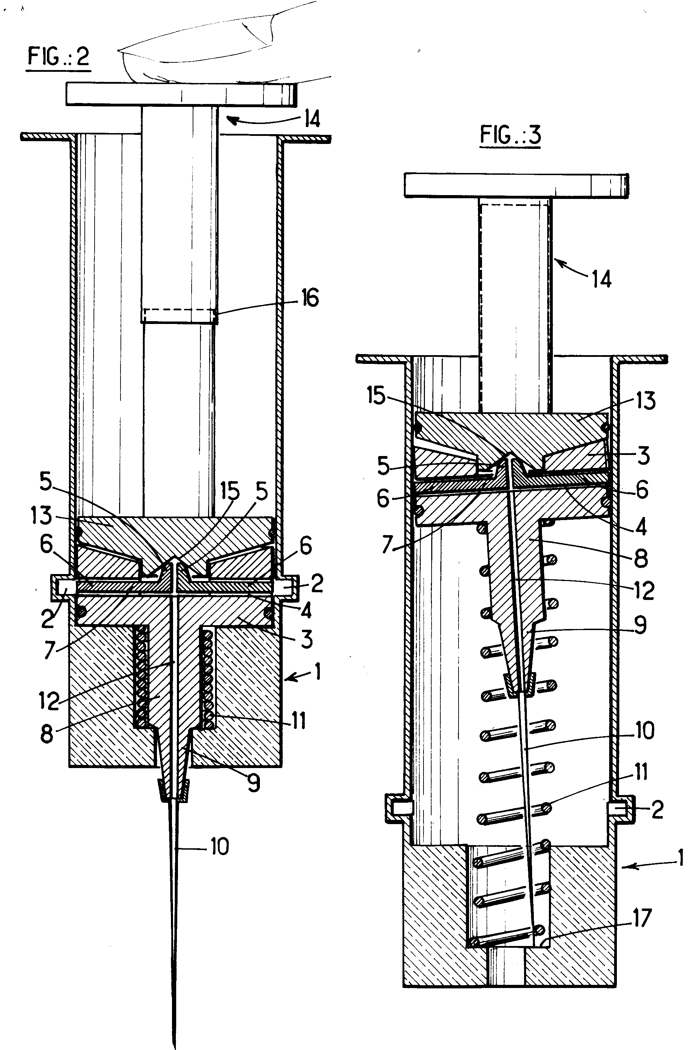

The apparatus thus presented (Fig. 1) is ready to function; after a needle has been put in place, the solution has been drawn up, and air purged by inversion, the injection is performed by pressing on the pusher; at the end of injection (Fig. 2), when the cam 15 comes to bear on the heads 5 of the bolts 6, it provokes the centripetal translation of said bolts, which are disengaged from the recesses 2, releasing the movable base 3 which rises, under the relaxation of the spring 11, pushing the plunger 13 upward and pulling the needle inside the body 1 (Fig. 3).

When the needle 10 is completely inside the body 1, it is deviated from the axis of the body by the spring 11. By pushing again the pusher 14, the needle engages the bottom 17 of the body. By further pushing the pusher 14, the frangible parts 16 are broken, and the pusher is shortened as shown in Figure 3.

According to an alternative which is not illustrated, and which does not include a spring 11, the retraction of the movable base 3 can be effected by simple traction on the plunger, in which case the external circumference of the cam 15 locks on the movable base 3 by means of a clip system; the pusher 14 is then broken level with the body, at a zone of lesser strength.

## Claims

1. A device for parenteral injections, comprising a body in which a plunger moves, and a needle which retracts automatically and completely within the body at the end of the injection when the plunger arrives at the base of the body, comprising a movable base which is integral with the locking end-piece of the needle and is blocked by one or more bolts engaged in a recess in the wall of the body, a spring held compressed on the lower face of the movable base, the abutment of the plunger against the upper face of the movable base provoking the release of the bolt or bolts, by displacement of a cam surface, and the unblocking of the movable base, so that the spring can then push back the movable base and the plunger, with the needle coming completely within the body, wherein the plunger bears said cam, which acts directly on the bolt or bolts.

2. The device as claimed in claim 1, wherein the cam borne by the plunger is constructed as a "flat" cylinder, the cross section of which bears a conical recess such that all its longitudinal sections present a cross section in the shape of a spread-out W, in such a manner that its bearing on the head of the bolts, which are constructed in the shape of an L lying on its side, provokes the mobilization of said bolts.

3. The device as claimed in claim 2, representing a tool for the manufacture of the device as claimed in claim 1, wherein it consists of a plunger equipped with a cam which is of conical shape and acts by spreading the bolts, permitting the charging of the device by introduction of the movable base into the body and the engagement of the bolts in the recesses following compression of the spring.

4. The device as claimed in claim 1, wherein it comprises a plunger pusher in two parts of approximately equal length and complementary cross section, allowing them to slide one within the other, these parts being joined near their ends by a connection of low strength (wedge, pin, punctiform fusion or bonding) in order to constitute a pusher which is twice the length of its elements and which is capable of breaking, under a given force exerted in the longitudinal direction, in order to reduce its length by half after use.

5. The device as claimed in claim 1, wherein the pusher is constructed in two parts, the one sliding in the other, these parts being integral during the injection by means of a connection which can be broken by a pressure on the pusher slightly greater than that which is necessary for the release of the bolts, in such a way that the needle can be retracted without the pusher rising.

6. The device as claimed in claim 1, wherein the spring which pushes the movable base bears on at least one of its end seats at a point which is sufficiently eccentric in relation to the axis of the needle to ensure that, when this spring is compressed after having been released, it assumes the general shape of an arc of a circle and deflects the needle from the axial direction, the deflection being sufficient to ensure that the needle cannot leave the body of the device through the orifice via which it entered.
